# EUROPÄISCHE PATENTSCHRIFT

(11) **EP 3 569 148 B1**
(45) Veröffentlichungstag und Bekanntmachung des Hinweises auf die Patenterteilung: **11.01.2023**
(21) Anmeldenummer: 18172938.5
(22) Anmeldetag: 17.05.2018
(51) Int. Cl.: A61B 6/00, G01N 23/06

(54) **VERFAHREN ZUR AUFNAHME VON EINEM BILDDATENSATZ MIT EINEM RÖNTGENDETEKTOR**
METHOD OF RECORDING AN IMAGE DATA SET WITH A X-RAY DETECTOR
PROCÉDÉ D'ENREGISTREMENT D'UN ENSEMBLE DE DONNÉES D'IMAGE AU MOYEN D'UN DÉTECTEUR DE RAYONS X

(43) Veröffentlichungstag der Anmeldung: 20.11.2019
(73) Patentinhaber: Siemens Healthcare GmbH, 91052 Erlangen (DE)
(72) Erfinder: Beister, Marcel, 91058 Erlangen (DE); Ergler, Thorsten, 91054 Erlangen (DE); Hupfer, Martin, 91052 Erlangen (DE); Kappler, Steffen, 91090 Effeltrich (DE); Köster, Niko, 91077 Neunkirchen am Brand (DE)

(56) Entgegenhaltungen:
- EP-A2- 1 598 663
- WO-A1-2015/014525
- JP-A- 2015 116 408

## Beschreibung

Die Erfindung betrifft ein Verfahren, eine Röntgenquelle, ein medizinisches Gerät, eine Verwendung des medizinischen Geräts, ein Computerprogrammprodukt und ein computerlesbares Medium, welche die Bereitstellung eines im Wesentlichen lückenlosen Bilddatensatzes ermöglichen.

In der Röntgenbildgebung, beispielsweise in der Radiographie, der Computertomographie oder der Angiographie können zählende direkt-konvertierende Röntgendetektoren oder integrierende indirekt-konvertierende Röntgendetektoren verwendet werden.

Die Röntgenstrahlung oder die Photonen können in direktkonvertierenden Röntgendetektoren durch ein geeignetes Konvertermaterial in elektrische Pulse umgewandelt werden. Als Konvertermaterial können beispielsweise CdTe, CZT, CdZnTeSe, CdTeSe, CdMnTe, InP, TlBr2, HgI2, GaAs oder andere verwendet werden. Die elektrischen Pulse werden von einer Auswerteelektronik, beispielsweise einem integrierten Schaltkreis (Application Specific Integrated Circuit, ASIC), bewertet. In zählenden Röntgendetektoren wird einfallende Röntgenstrahlung durch Zählen der elektrischen Pulse, welche durch die Absorption von Röntgenphotonen im Konvertermaterial ausgelöst werden, gemessen. Die Höhe des elektrischen Pulses ist in der Regel proportional zur Energie des absorbierten Röntgenphotons. Dadurch kann eine spektrale Information durch den Vergleich der Höhe des elektrischen Pulses mit einem Schwellwert extrahiert werden.

Die Röntgenstrahlung oder die Photonen können in indirekt-konvertierenden Röntgendetektoren durch ein geeignetes Konvertermaterial in Licht und mittels Photodioden in elektrische Pulse umgewandelt werden. Als Konvertermaterial werden häufig Szintillatoren, beispielsweise GOS (Gd2O2S), CsJ, YGO oder LuTAG, eingesetzt. Szintillatoren werden insbesondere in der medizinischen Röntgenbildgebung im Energiebereich bis 1MeV eingesetzt. Üblicherweise werden sogenannte indirekt-konvertierende Röntgendetektoren, sogenannte Szintillatordetektoren, verwendet, bei denen die Konvertierung der Röntgen- oder Gammastrahlen in elektrische Signale in zwei Stufen erfolgt. In einer ersten Stufe werden die Röntgen- oder Gammaquanten in einem Szintillatorelement absorbiert und in optisch sichtbares Licht umgewandelt, dieser Effekt wird Lumineszenz genannt. Das durch Lumineszenz angeregte Licht wird anschließend in einer zweiten Stufe durch eine mit dem Szintillatorelement optisch gekoppelten ersten Photodiode in ein elektrisches Signal umgewandelt, über eine Auswerte- oder Ausleseelektronik ausgelesen und anschließend an eine Recheneinheit weitergeleitet.

Defekte Detektorpixel können in den Röntgenaufnahmen durch Interpolation von Umgebungspixeln maskiert werden. Diese Interpolation kann mit unterschiedlichsten Methoden durchgeführt werden. Der Informationsverlust lässt sich dadurch jedoch nicht kompensieren, sondern es handelt sich ausschließlich um eine kosmetische Korrektur, d.h. ohne korrekte Wiederherstellung der Information.

Ein Röntgendetektor und insbesondere ein Röntgen-Flachdetektor bestehen aus einer sehr großen Anzahl an Detektorelementen, welche auch als Pixel, Makropixel oder Subpixel bezeichnet werden können, wobei mehrere Subpixel ein Makropixel bilden. Eine Detektionsfläche wird aus einer Vielzahl von Detektorelementen gebildet. Typischerweise kann ein Teil dieser Detektorelemente defekt oder/und unbrauchbar für die klinische Bildgebung sein. Diese defekten oder unbrauchbaren Detektorelemente können als Defektpixel bezeichnet werden. Die Defekte können sowohl fertigungs- wie auch alterungsbedingt sein.

Aus der Druckschrift DE 196 17 126 A1 ist ein Computertomograph mit einer in z-Richtung sich erstreckenden Anode des Röntgenstrahlers bekannt, bei dem in einfacher Weise eine Fokusverstellung auf der Anode erfolgt. Der Röntgenstrahler enthält eine Reihe von Kickspulen, die nacheinander geschaltet werden, so dass ein magnetisches Wanderfeld entsteht, das den Fokus verstellt. Der Röntgenstrahler wird von Blenden zur Einblendung des Röntgenstrahlenbündels umgeben, von denen eine synchron mit der Fokusbewegung rotiert.

Aus der Druckschrift DE 196 39 920 A1 ist eine Röntgenröhre mit einem evakuierten Gehäuse, in dem fest damit verbunden eine Elektronen emittierende Kathode und eine über eine Antriebsvorrichtung drehbare Drehanode mit einem Anodenteller, auf die der mittels eines elektrischen Feldes beschleunigte Elektronenstrahl trifft, angeordnet sind, und mit einem elektromagnetischen System zum Ablenken und Fokussieren des Elektronenstrahls mit mehreren stromdurchflossenen Spulenelementen, wobei die Kathode einen rotationssymmetrischen Rundstrahl erzeugt, die Drehachse des Anodentellers parallel gegenüber der Achse des Elektronenstrahls um den mittleren Radius des Anodentellerrandes versetzt ist, und das elektromagnetische System ein den Strahlquerschnitt verformendes Dipolfreies Quadrupolfeld erzeugt.

Aus der Druckschrift JP 2015 116408 A ist eine Röntgen-CT-Vorrichtung und ein Korrekturverfahren für ein defektes Element bekannt, das einen Wert eines defekten Elements in einem Röntgendetektor korrigieren kann. Ein Röntgenstrahl, der auf ein normales Element in der Nähe eines defekten Elements mit einem Strahldurchgang auftrifft, der im Wesentlichen einem Strahldurchgang eines auf ein defektes Element auftreffenden Röntgenstrahls entspricht, wird durch Röntgenfokusverschiebung in einer Röntgenröhrenvorrichtung erzeugt, und ein Ausgangswert des defekten Elements wird durch Verwendung eines Ausgangswerts des normalen Elements korrigiert. Eine Bildverarbeitungsvorrichtung erfasst übertragene Röntgendaten mit Röntgenstrahlen, die aus einer Vielzahl von Röntgenbrennpositionen bestrahlt werden, und ersetzt den Ausgangswert des defekten Elements durch den Ausgangswert des normalen Elements in der Nähe des defekten Elements mit den aus einer zweiten Brennposition bestrahlten Röntgenstrahlen, wenn Projektionsdaten auf der Grundlage der übertragenen Röntgendaten mit den aus einer ersten Brennposition bestrahlten Röntgenstrahlen erzeugt werden.

Der Erfindung liegt das Problem zugrunde, dass ab einer vorbestimmten Anzahl defekter Detektorelemente oder einem Überschreiten einer vorbestimmten Dichte von defekten Detektorelementen pro Fläche, beispielsweise in einem kleinen begrenzten Bereich, der Röntgendetektor bei Neusystemen nicht verwendet werden kann bzw. bei laufenden Systemen getauscht werden sollte.

Es ist Aufgabe der Erfindung, ein Verfahren, eine Röntgenquelle, ein medizinisches Gerät, eine Verwendung des medizinischen Geräts, ein Computerprogrammprodukt und ein computerlesbares Medium anzugeben, welche die Bereitstellung eines im Wesentlichen lückenlosen Bilddatensatzes ermöglichen.

Die Aufgabe wird erfindungsgemäß gelöst durch ein Verfahren nach Anspruch 1, eine Röntgenquelle nach Anspruch 13, ein medizinisches Gerät nach Anspruch 14, eine Verwendung des medizinischen Geräts nach Anspruch 15, ein Computerprogrammprodukt nach Anspruch 16 und ein computerlesbares Medium nach Anspruch 17.

Die Erfindung betrifft ein Verfahren zur Aufnahme von einem Bilddatensatz mit einem Röntgendetektor umfassend eine Detektionsfläche mit einer Vielzahl von Detektorelementen und einer Röntgenquelle mit einem Brennfleck. Das Verfahren umfasst die Schritte, bevorzugt in der folgenden Reihenfolge, des erstens Erfassens, des Verschiebens, des zweiten Erfassens und der Kombination. Im Schritt des ersten Erfassens wird eine erste Aufnahme mit einer ersten Position des Brennflecks erfasst bzw. aufgenommen. Im Schritt des Verschiebens wird der Brennfleck von der ersten Position zu einer von der ersten Position verschiedenen zweiten Position des Brennflecks um einen Abstand verschoben. Im Schritt des zweiten Erfassens wird eine zweite Aufnahme mit der zweiten Position erfasst bzw. aufgenommen. Im Schritt der Kombination werden die erste Aufnahme und die zweite Aufnahme zum Bilddatensatz kombiniert.

Der Bilddatensatz kann einen sogenannten Rohdatensatz oder einen bereits (vor-)verarbeiteten Datensatz mit einzelnen Bildwerten umfassen. Die Bildwerte können üblicherweise in eine Matrix angeordnet sein. Die Bildwerte können Intensitäts-, Energie- und/oder Zählwerte darstellen. Auf dem Bilddatensatz kann ein, insbesondere vollständiges, Bild basieren bzw. erzeugt werden. Das Bild kann zwei-, drei- oder vierdimensional sein.

Der Brennfleck kann insbesondere der sogenannte optische Brennfleck sein. Das Verschieben des Brennflecks erfolgt insbesondere bezogen auf die Detektionsfläche. Das Verschieben des Brennflecks kann entlang der x- oder y-Richtung des Röntgendetektors oder entlang einer beliebigen, bevorzugt geraden, Trajektorie innerhalb der Detektionsfläche erfolgen. Das Verschieben des Brennflecks kann alternativ oder zusätzlich entlang einer Bewegungsrichtung des Röntgenquellen-Detektor-Systems oder der Röntgenquelle an sich erfolgen, beispielsweise bei Aufnahmen umfassend eine Bewegung oder Rotation zumindest des Röntgendetektors oder der Röntgenquelle, insbesondere um drei- oder vierdimensionale Bilder zu erzeugen oder/und eine relativ zur Ausdehnung der Detektionsfläche große räumliche Veränderung aufzunehmen. Die Detektionsfläche kann insbesondere flächig und eben ausgebildet sein. Sowohl die erste Position als auch die zweite Position können sich bevorzugt in der Ebene der Detektionsfläche befinden. Der Abstand kann sich entsprechend auf einen Abstand innerhalb der Detektionsfläche beziehen. Die zweite Position bzw. der Abstand kann vorbestimmt oder zufällig gewählt sein. Das Verschieben kann auch als Verändern bezeichnet werden. Das Verschieben bzw. Verändern kann in Form eines Wechsels zwischen verschiedenen Foki bzw. Brennflecken, beispielsweise der Röntgenquelle oder verschiedener Röntgenquellen, erfolgen.

Die verschiedenen Brennflecke können als erster Brennfleck mit der ersten Position und als zweiter Brennfleck mit der zweiten Position ausgebildet sein.

Die Röntgenquelle sendet in der Regel einen Zentralstrahl aus, welcher bevorzugt parallel zu einer Flächennormale der Detektionsfläche verläuft. Die Flächennormale kann insbesondere mittig auf der Detektionsfläche festgelegt sein.

Das Verfahren kann um weitere Schritte des Verschiebens und des zweiten Erfassens erweitert werden, d.h. das Verfahren kann die Schritte des weiteren Verschiebens von der zweiten Position des Brennflecks zu einer weiteren Position des Brennflecks und des weiteren Erfassens einer weiteren Aufnahme mit der weiteren Position umfassen. Die weitere Position kann von der ersten Position und/oder zweiten Position verschieden sein. Das Verfahren kann Wiederholung der Verfahrensschritte umfassen, beispielsweise eine alternierendes oder zyklisches Verschieben des Brennflecks.

Springfokusröhren können in der Volumen- oder Schichtbildgebung, beispielsweise CT-Bildgebung, verwendet werden, um die Bildqualität der Aufnahmen zu steigern. Eine Springfokusröhre ist eine Röntgenröhre, bei der der Röntgenfokus bzw. der elektronische Brennfleck auf der Anode, insbesondere dem Anodenteller, zwischen einzelnen Röntgenaufnahmen verschoben wird. Die Verschiebung ist sowohl in Rotationsrichtung als auch senkrecht zur Rotationsrichtung oder gleichzeitig in beide Richtungen möglich. Die Verschiebung des elektronischen Brennflecks führt in der Regel zu einer Verschiebung des optischen Brennflecks um einen Abstand d, welcher die Verschiebung des optischen Brennflecks in der Bildebene angibt. Dadurch werden Details des zu untersuchenden Objektes auf unterschiedlichen Bereichen auf der Detektionsfläche abgebildet.

Erfindungsgemäß kann die Verschiebung des Brennflecks dazu genutzt werden, um die Totbereiche des Röntgendetektors, also defekte oder unbrauchbare Detektorelement oder Lücken beispielsweise in Form von Fugen, auszugleichen. Die Erfindung kann sowohl im Bereich der 2D-, als auch der 3D- oder 4D-Röntgenbildgebung angewendet werden. Im Fall der 2D-Bildgebung werden statt einer einzigen Röntgenaufnahme zwei oder mehr Aufnahmen in kurzer Abfolge durchgeführt. Zwischen den Aufnahmen springt der Brennfleck auf dem Anodenteller bzw. wird der optische Brennfleck um einen Abstand verschoben. Im Fall der 3D-Bildgebung wird der optische Brennfleck zwischen einzelnen Projektionsaufnahmen verschoben. Im Fall der 4D-Bildgebung wird die 3D-Bildgebung um die Dimension der Zeit erweitert, so dass ein zeitlicher Verlauf von Schwächungswerten, beispielsweise basierend auf der Verwendung von einem Kontrastmittel oder einem Marker, mittels der 4D-Bildgebung abgebildet werden kann.

Der Abstand kann bevorzugt so gewählt werden, dass die Verschiebung der relevanten Details des zu untersuchenden Objektes einige Detektorelemente beträgt. Dadurch werden diese Details des Objekts, welche von der ersten Aufnahme beispielsweise durch einen oder mehrere defekte Detektorelemente bzw. eine Fuge nicht umfasst sind, in einer zweiten bzw. n-ten Aufnahme erfasst. Nur in seltenen Fällen können an den entsprechenden Lagen innerhalb der folgenden Aufnahme oder den folgenden Aufnahmen auch jeweils defekte Detektorelemente erwartet werden. Defekte Detektorelemente können vor der ersten Aufnahme bestimmt werden, damit kann der Abstand derart gewählt werden, dass das Detail des zu untersuchenden Objekts an einer anderen Lage in der folgenden Aufnahme zu finden ist, wobei diese andere Lage einem funktionsfähigen Bereich der Detektionsfläche entspricht. Nicht-sensitive Bereiche, beispielsweise Fugen zwischen etwaigen Detektorkacheln, sind a priori bekannt. Die Sprungdistanz kann entsprechend so vorbestimmt werden, dass die Details des zu untersuchenden Objekts bestmöglich durch funktionsfähige Detektorelemente abgedeckt werden.

Der Röntgendetektor kann zur Detektion jeglicher Strahlung ausgebildet sein, die eine Bildgebung ermöglicht, also beispielsweise geeignete Teilchenstrahlung (Elektronen, Positronen, Alpha-Teilchen etc.) und/oder elektromagnetische Strahlung (Photonen). Bevorzugt ist der Röntgendetektor zur Detektion von Röntgenphotonen in einem zur medizinischen Bildgebung geeigneten Energiebereich, beispielsweise in einem Energiebereich innerhalb des Bereichs von 10keV bis 200keV, ausgebildet. Dazu kann die Strahlung zwischen den Aufnahmen durchgehend oder auch für die jeweilige Aufnahme erzeugt auf die Detektionsfläche des Röntgendetektors auftreffen, sodass bis auf die Verschiebung des Brennflecks im Wesentlichen gleiche Aufnahmen erfasst werden. Bevorzugt handelt es sich bei dem Röntgendetektor um einen Röntgen-Flachdetektor. Die Detektionsfläche bezeichnet dabei eine Fläche, die zur Detektion der Strahlung ausgebildet ist, die also für die Strahlung sensitiv ist und sie quantitativ, beispielsweise in Form von Intensitäts-, Zähl- und/oder Energiewerten, erfasst. Beispiele für die Detektionsfläche sind die Oberflächen von direkt-konvertierenden Röntgendetektoren (CdTe, CZT, CdZnTeSe, CdTeSe, CdMnTe, InP, TlBr2, HgI2, GaAs, a-Se oder andere), indirekt-konvertierenden Festkörperröntgendetektoren mit einem Szintillator-Konverterelement, CMOS-Sensoren oder dergleichen. Bei der Oberfläche kann es sich beispielsweise auch um eine gewölbte bzw. gekrümmte Fläche handeln, bevorzugt ist die Detektionsfläche jedoch eben bzw. plan.

Die Aufnahmen bezeichnen die vom Röntgendetektor über einen definierten (kurzen) Zeitabschnitt, beispielsweise im Bereich von einigen Millisekunden, pixelweise quantitativ erfasste Strahlung, also die Detektion der Strahlung als Messwerte ihrer Intensität oder auch anderer Größen wie zum Beispiel der Wellenlänge bzw. Energie oder der Anzahl der Photonen. Bei einer derart erfassten Aufnahme kann sich beispielsweise um eine Projektion eines Untersuchungsobjekts bei einer Röntgenaufnahme handeln. Bei herkömmlichen Verfahren würde also die Aufnahme auch direkt dem zu erzeugenden Bild entsprechen. Demgegenüber werden erfindungsgemäß mehrere Aufnahmen, insbesondere die erste Aufnahme und die zweite Aufnahme, zu einem Bilddatensatz bzw. einem Bild kombiniert.

Die Detektorelemente bzw. die Pixel sind in der Regel die kleinsten auslesbaren Einheiten des Röntgendetektors, aus denen sich die Detektionsfläche zusammensetzt. Die Detektorelemente können in Form von Subpixeln zu Pixelgruppen in Form von Makropixeln zusammengefasst werden. Die Detektorelemente sind beispielsweise als Feld, als Raster bzw. als Matrix mit einer Vielzahl von Zeilen und/oder Spalten, in der Regel in einer orthogonalen Anordnung, angeordnet. Beim Auslesen wird den Detektorelementen - beispielsweise elektronisch - ein Messwerte, beispielsweise Intensitäts-, Energie- und/oder Zählwert, in Abhängigkeit von der detektierten Strahlungsintensität bzw. der deponierten Strahlungsmenge oder/und der Strahlungsenergie zugeordnet.

Zwischen den Aufnahmen wird zumindest der Brennfleck der Röntgenquelle jeweils um einen definierten Abstand verschoben, der bevorzugt größer ist als eine Erstreckung eines Detektorelements. Alternativ kann auch zu einem anderen Brennfleck der Röntgenquelle oder einem Brennfleck einer weiteren Röntgenquelle gewechselt werden. Die Verschiebung des Brennflecks erfolgt dabei beispielsweise in Relation zum Röntgendetektor und/oder zu einem aufzunehmenden Objekt. Somit sind also auch die Aufnahmen im Verhältnis zueinander verschoben. Die Verschiebung erfolgt dabei in der Regel in einer Richtung der Detektionsfläche des Röntgendetektors. Bei einem ebenen bzw. planen Röntgendetektor kann die Verschiebung dementsprechend in jeder Richtung der Ebene erfolgen, also beispielsweise in Richtung der Zeilen oder der Spalten oder auch schräg dazu. Bei einer gekrümmten Fläche kann die Verschiebung sowohl eine Rotation in Krümmungsrichtung als auch eine Translation senkrecht dazu umfassen. Die Verschiebung ist in ihrer Distanz gleich den oder größer als die Abmessungen bzw. Erstreckungen eines Detektorelements. Die Position und Ausdehnung eines Detektorelements überlappt bevorzugt nicht zwischen zwei Aufnahmen. Dadurch wird die durch die Strahlung bzw. deren Intensität oder Energie charakterisierte Bildinformation zu einer Objektregion bei jeder Aufnahme auf ein anderes Detektorelement des Röntgendetektors abgebildet.

Wenn also beispielsweise in einer Position der Detektionsfläche im Rahmen einer ersten Aufnahme die beispielsweise auf ein defektes Detektorelement oder einen Spalt zwischen benachbarten Detektorelementen abgebildete Bildinformation nicht korrekt detektiert wird, wird für eine weitere Aufnahme durch die Verschiebung der Detektionsfläche die Objektregion auf eine Position eines funktionsfähigen Detektorelements bewegt. Dadurch kann ein die Bildinformation repräsentierender Messwert korrekt ausgelesen werden. Somit lässt sich vorteilhafterweise trotz des defekten Detektorelements bzw. des Spalts zwischen benachbarten Detektorelement auch an dieser Position ein korrekter Bildpunktwert bzw. Messwert ermitteln. Im Gegensatz zum Stand der Technik kommt es also erfindungsgemäß zu keinem Informationsverlust.

Für einen Bildpunkt des Bilddatensatzes wird aus den jeweiligen Detektorelementen der Aufnahmen, d. h. bevorzugt aus dem Bildpunkt bzw. der Objektregion zugeordneten Detektorelementen, mittels der Kombination von Detektorelementen aus den unterschiedlichen Aufnahmen aus den jeweiligen Messwerten ein Bildpunktwert ermittelt. Unter der Kombination der Aufnahmen wird grundsätzlich jegliches Zusammenführen der von den Aufnahmen umfassten Messwerte bzw. Rohdaten verstanden. Die Aufnahmen können also insgesamt, pixelweise (d.h. detektorelementweise) oder in Gruppen von Detektorelementen, d.h. bereichsweise, kombiniert werden. Die Kombination kann zum Beispiel eine Linearkombination oder eine nichtlineare Kombination der Messwerte sein. Würde also für einen Bildpunkt aufgrund der Zuordnung ein defektes Detektorelement einer Aufnahme oder eine durch einen Spalt zwischen benachbarten Detektorelementen fehlende Bildinformation in die Kombination miteinfließen, könnte die Kombination beispielsweise ausschließlich auf Basis der funktionstüchtigen Detektorelements der anderen Aufnahmen erfolgen. Durch die Erfindung kann somit eine deutlich höhere Anzahl an defekten Detektorelementen oder höhere Anzahl oder größere Breite der Spalte (d.h. Lücken) zwischen benachbarten Detektorelementen toleriert werden.

Grundsätzlich genügen bereits zwei Aufnahmen zur Erzeugung eines Bilddatensatzes mit Hilfe des erfindungsgemäßen Verfahrens. Je nach den Anforderungen können allerdings auch beliebig viele weitere Aufnahmen in verschiedenen Positionen, also mit unterschiedlichen Verschiebungen, erfasst werden, indem die Schritte des Verschiebens und der anschließenden Aufnahme je nach Bedarf wiederholt werden. Das Bild bzw. der Bilddatensatz wird dabei als Produkt bzw. Ergebnis des erfindungsgemäßen Verfahrens verstanden. Je nach Art und Weise der Kombination kann es beispielsweise eine möglichst getreue Abbildung eines darzustellenden Objektes bzw. einer Objektregion wiedergeben oder es können darin bestimmte Details hervorgehoben und so besser kenntlich gemacht werden, wie später noch näher erläutert wird.

Vorteilhaft kann eine deutlich höhere Anzahl an defekten Detektorelementen toleriert werden. Zudem bestehen manche Röntgendetektoren aus mehreren Segmenten bzw. "Kacheln" von Detektorelementen die zur gesamten aktiven Fläche zusammengesetzt werden. Ein typisches Beispiel hierfür sind photonenzählende Detektoren aufweisend mindestens ein CdTe-Konverterelement oder CMOS-Röntgendetektoren. Die Fugen bzw. die Spalte zwischen den Kacheln sind nicht sensitiv für Röntgenstrahlen und damit "tote" bzw. nicht-sensitive Bereiche. Vorteilhaft können die vormals verlorenen Bilddaten erzeugt werden.

Bei einem interpolations-basierten Korrekturverfahren werden defekte Pixel lediglich maskiert und nicht wiederhergestellt, d.h. die Informationen bleiben weiterhin verloren. Durch das Verschieben des Brennflecks wird mit hoher Wahrscheinlichkeit jedes Detail der Objektregion, welches zuvor in einem defekten Bereich oder einem Spalt abgebildet wurde, nun in einem funktionierenden Pixel abgebildet. Im Gegensatz zur Interpolation kann der Defekt bzw. fehlende Messwerte nun, in der 2D-, 3D- und 4D-Bildgebung, durch einen real gemessenen Messwert ausgeglichen werden. Daher kann eine deutlich höhere Zahl an defekten Detektorelementen toleriert werden, was mehrere Vorteile mit sich bringt. Vorteilhaft können Röntgendetektoren mit einer höheren Anzahl an Defekten verwendet werden. Vorteilhaft können Röntgendetektoren, welche durch Alterung eine höhere Anzahl an Defekten aufweisen, zu einem späteren Zeitraum getauscht werden. Dies reduziert vorteilhaft die Lebensdauerkosten. Vorteilhaft können Röntgendetektoren, welche bauart-bedingt zu viele tote Detektorelemente für Anwendungen mit sehr hohen Qualitätsanforderungen haben, beispielsweise photonenzählende Röntgendetektoren aus vielen einzelnen Kacheln, insbesondere für die Mammographie, mit der vorliegenden Erfindung für diese Anwendung verwendet werden. Damit können sich vorteilhaft neue Möglichkeiten und Anwendungen, wie etwa spektrale Bildgebung sowie dosiseffizientere Nutzung der Röntgenstrahlung, erreicht bzw. ermöglicht werden.

Neben der Korrektur von defekten Detektorelementen sind weitere Vorteile durch die vorgeschlagene Erfindung möglich. Die Erhöhung der Ortsauflösung kann durch Versatz um ein ganzzahlig-Vielfaches plus ein halbes Detektorelement ermöglicht werden. Damit kann die Abtastfrequenz erhöht werden. Es kann vorteilhaft eine Reduktion von spezifischen Artefakten (z.B. Aliasing-Artefakte) durch die verbesserte Abtastung erreicht werden.

Gemäß einem Aspekt der Erfindung beträgt der Abstand in der Projektion auf die Detektionsfläche um n oder n+0,5 Detektorelemente, wobei n eine natürliche Zahl ist. Bevorzugt beträgt der Abstand 1, 1,5, 2,5 oder 3,5 Detektorelemente.

Der Brennfleck wird in einer Variante des erfindungsgemäßen Verfahrens bevorzugt um n+0,5 Detektorelemente verschoben. Dabei ist n eine natürliche Zahl, die bevorzugt größer als 0 ist. Durch die Verschiebung um mehr als ein Detektorelement können einerseits durch defekte Detektorelemente erzeugte Artefakte bereinigt werden. Andererseits kann mittels der zusätzlichen Verschiebung um ein halbes Detektorelement zugleich vorteilhafterweise die Auflösung des Bildes erhöht werden, wie im Folgenden beschrieben wird. Bei der Kombination der Aufnahmen können Messwerte ein oder auch mehrere Detektorelemente einer Aufnahme, die im Bereich des zu erzeugenden Bildpunktes angeordnet sind, zur Ermittlung eines Bildpunktwertes verwendet werden. Zum Beispiel wird durch die Verschiebung des Brennflecks um etwa 1,5 Detektorelemente in einer Richtung des Rasters bzw. in einer diagonalen Richtung in der zweiten Aufnahme der Brennfleck mittig zwischen zwei bzw. vier Detektorelementen der ersten Aufnahme angeordnet. Dadurch ergibt sich bei einer Kombination, also beispielsweise bei einem Übereinanderlegen der Aufnahmen, ein feineres, d.h. doppelt so feines bzw. viermal so feines, Raster der Bildpunkte als bei dem Raster der Detektorelemente der ursprünglichen Aufnahmen. Für die zweite Aufnahme verläuft die Projektion entlang eines anderen Pfads von der Röntgenquelle durch das Objekt zur Detektionsfläche im Vergleich zur ersten Aufnahme. Der Messwert des Detektorelements der zweiten Aufnahme wird mit dem Messwert des ihm jeweils zugeordneten Detektorelements der ersten Aufnahme kombiniert, sodass insbesondere die flächige Schnittmenge des Detektorelements der ersten Aufnahme mit dem Detektorelement der zweiten Aufnahme doppelt gewichtet wird.

Besonders bevorzugt erfolgt die Verschiebung um etwa 1,5 Detektorelemente. Dies ist die kürzeste Distanz, bei der die defekten Pixel ausgeglichen werden können und zugleich auch eine höhere Auflösung erzielt werden kann.

Bevorzugt findet die Verschiebung des Brennpunkts diagonal statt, also beispielsweise zu gleichen Teilen sowohl in Richtung der Zeilen als auch der Spalten der Matrix bzw. des Rasters. Dadurch kann vorteilhafterweise die Auflösung in diesen beiden Richtungen, insbesondere gleichzeitig, erhöht werden.

Erfindungsgemäß ist der Röntgendetektor in Detektorsegmente mit jeweils einer Anzahl von Detektorelementen unterteilt und zwischen den Detektorsegmenten sind Fugen mit einer Fugenbreite angeordnet. Erfindungsgemäß wird der Abstand in der Projektion auf die Detektionsfläche um zumindest eine Fugenbreite verschoben. Eine Fuge kann auch als Spalt bezeichnet werden.

Der Röntgendetektor kann eine Anzahl von Detektorsegmenten, zwischen denen Fugen mit einer Fugenbreite angeordnet sind, umfassen. Der Brennfleck wird daher vorzugsweise um zumindest eine Fugenbreite verschoben. Wenn die Fugen zwischen den Detektorsegmenten sowohl in einer Zeilenrichtung als auch in einer Spaltenrichtung angeordnet sind, erfolgt die Verschiebung bzw. das Verschieben bevorzugt auch zugleich um mindestens eine Fugenbreite in Zeilenrichtung und in Spaltenrichtung. Der Abstand zwischen zwei Detektorsegmenten, also die Fugenbreite, beträgt beispielsweise ca. 400 µm, was üblicherweise der Ausdehnung von 5 bis 10 Pixeln entsprechen kann. Vorteilhaft können somit nicht nur die Effekte einzelner defekter Detektorelemente korrigiert bzw. entfernt werden, sondern mittels der Kombination zugleich auch lückenlos Bildwerte im Bereich der Fugen ermittelt werden, was bei einer herkömmlichen Aufnahme nicht möglich wäre.

Gemäß einem Aspekt der Erfindung wird der Abstand basierend auf einer Clustergröße von defekten Detektorelementen bestimmt. Die Clustergröße kann bevorzugt eine maximale Clustergröße bezeichnen. Die maximale Clustergröße kann den größten zusammenhängenden Bereich von defekten Detektorelementen innerhalb eines Röntgendetektors oder eines Detektorsegments angeben. Die Clustergröße kann beispielsweise in einer Anzahl von benachbarten defekten Detektorelementen angegeben werden. Die benachbarten defekten Detektorelemente können insbesondere zusammenhängend bzw. lückenlos angeordnet sein. Die Clustergröße kann in einer Erstreckung, beispielsweise entlang der Zeilen- oder/und Spaltenrichtung, der benachbarten defekten Detektorelemente angegeben werden.

Die Clustergröße kann in einem Schritt des Bestimmens bestimmt werden. Der Schritt des Bestimmens kann vorteilhaft vor dem ersten Erfassen durchgeführt werden. Beispielsweise kann dabei eine Funktionsprüfung der Detektorelemente erfolgen. Beispielsweise kann die Funktionsprüfung direkt nach der Fertigung vom Hersteller oder mittels einer Testmessung bzw. einer Kalibrierungsmessung des Strahlungsdetektors und mittels einer Analyse der Bilddaten (also Abweichungen der detektierten Intensitäten) vor der ersten Aufnahme bestimmt werden. Die Funktionsprüfung kann eine elektrische oder elektronische Funktionsprüfung umfassen. Die Funktionsprüfung kann die Verwendung von elektromagnetischen Strahlen, beispielsweise infrarotem oder sichtbarem Licht oder Röntgenstrahlung, umfassen. Beispielsweise kann eine im Wesentlichen gleichmäßige Beleuchtung des Röntgendetektors mittels der Röntgenquelle ohne Untersuchungsobjekt im Strahlengang erfolgen. Ein Detektorelement kann als defekt klassifiziert werden, wenn es ein Qualitätskriterium unterschreitet bzw. nicht erfüllt. Das Qualitätskriterium kann ein Schwellwert sein. Das Qualitätskriterium kann ein logischer Wert, d.h. wahr/falsch, sein. Es kann mittels der Funktionsprüfung bzw. mittels deren Ergebnisses eine Defektmatrix bzw. eine Landkarte der defekten Detektorelemente erstellt werden. Die defekten Detektorelemente können dann beispielsweise in einem Gerätetreiber des Röntgendetektors, des medizinischen Geräts oder einer entfernten Verarbeitungseinheit vermerkt sein, sodass sie bei einer Steuerung der Verschiebung des Brennflecks und gegebenenfalls bei der folgenden Kombination berücksichtigt werden.

Es kann beispielsweise für eine Mehrzahl von, insbesondere defekten, Detektorelementen, bevorzugt für jedes Detektorelement, geprüft werden, ob mindestens ein benachbartes Detektorelement ebenfalls defekt ist. Es können damit defekte Bereiche oder Cluster in der Detektormatrix bestimmt werden. Die Anzahl der, insbesondere defekten Detektorelemente, innerhalb jedes Clusters kann bestimmt werden. Diese Anzahl kann die Clustergröße darstellen. Die maximale Clustergröße kann zur Bestimmung des Abstands herangezogen werden. Dadurch können vorteilhaft alle defekten Detektorelemente mittels des erfindungsgemäßen Verfahrens korrigiert werden. Es kann auch eine beispielsweise mittlere Clustergröße oder ein Schwerpunkt der Verteilung der Clustergrößen zur Bestimmung oder Auswahl des Abstands herangezogen werden. Dadurch kann vorteilhaft für eine Vielzahl von defekten Detektorelementen eine Korrektur erreicht werden. Zusätzlich kann eine Interpolation für nicht-korrigierte defekte Detektorelemente erfolgen.

Gemäß einem Aspekt der Erfindung ist eine Verschiebezeit des Brennflecks kürzer oder gleich einer Auslesezeit des Röntgendetektors. Eine Verschiebezeit des Fokuspunkts kann im Wesentlichen einer Auslesezeit des Röntgendetektors entsprechen.

Das heißt, dass die Zeit, die für das Verschieben des Brennflecks verwendet oder benötigt wird, im Wesentlichen der Zeit gleicht, die verwendet bzw. benötigt wird, um die einzelnen bzw. alle Detektorelemente des Röntgendetektors auszulesen. Dadurch kann der Brennfleck im Wesentlichen zeitgleich verschoben und der Röntgendetektor währenddessen ausgelesen werden. Vorteilhaft kann die Zeit für die Aufnahme des Bilddatensatzes verringert werden. Die Auslesezeit bzw. die Verschiebezeit betragen dabei besonders bevorzugt in etwa 100 ms. Beispielsweise können auch Artefakte, die durch die Bewegung eines Patienten während der bzw. zwischen den Aufnahmen entstehen, weitgehend vermieden werden. Bevorzugt kann der Brennfleck um wenige Detektorelemente verschoben werden. Die Verschiebezeit des Brennflecks kann gegenüber der Auslesezeit relativ kurz sein.

Gemäß einem Aspekt der Erfindung umfasst die Kombination der Aufnahmen eine pixelweise Mittelwertbildung und/oder eine pixelweise Maximalwertbildung.

Die Kombination der Aufnahmen kann vorzugsweise eine detektorelementweise Mittelwertbildung und/oder eine pixelweise Maximalwertbildung umfassen. Die erste und zweite Aufnahme werden insbesondere nicht als Ganzes miteinander kombiniert. Das kann heißen, dass für einen zu erzeugenden Bildpunkt die Messwerte der örtlich zugeordneten bzw. korrespondierenden Detektorelemente der unterschiedlichen Aufnahmen jeweils gemittelt und als Bildpunktwert verwendet werden. Das kann alternativ heißen, dass für einen zu erzeugenden Bildpunkt die Messwerte der örtlich zugeordneten bzw. korrespondierenden Detektorelemente der unterschiedlichen Aufnahmen nur der jeweils lokal maximale Mittelwert als Bildpunktwert verwendet wird. Die Kombination kann eine Mittelung, MIP, MEAN, ein Bildregistrierung oder nicht-rigide Transformation umfassen.

Die Maximalwertbildung kann in Bereichen der Aufnahmen ausgeführt werden, deren Messwerte über einem Schwellenwert liegen. Der Schwellwert kann insbesondere ein Messwert sein, der Weichgewebe bzw. Wasser von einer Kalzifikation bzw. Knochen abgrenzt. Vorteilhaft können sogenannte Hochkontrastbereiche zusätzlich noch hervorgehoben werden, was beispielsweise bei der mammographischen Bildgebung die Kenntlichkeit von Mikrokalzifikationen, die auf Tumore hindeuten, vorteilhaft erhöht und somit eine Diagnose erleichtert. Die Maximalwertbildung kann alternativ oder zusätzlich in Bereichen der Aufnahmen ausgeführt werden, die defekte Detektorelemente bzw. keine (korrekten) Messwerte aufweisen. In den übrigen Bereichen der Aufnahme, d.h. Bereiche ohne defekte Detektorelemente oder Bereiche mit Messwerten unterhalb des Schwellwerts, kann die Mittelwertbildung ausgeführt werden.

Bei der Verschiebung ist zu berücksichtigen, dass es gegebenenfalls auch zu räumlichen Ballungen von defekten Detektorelementen kommen kann. Bevorzugt ist die Verschiebung des Brennflecks in Bezug auf Richtung und Abstand so gestaltet, dass möglichst viele defekte Detektorelemente ausgeglichen werden können. Für defekte Detektorelemente einer Aufnahme können bevorzugt nur die jeweils andere Aufnahme bzw. die jeweils anderen Aufnahmen zur Kombination zu dem Bilddatensatz verwendet werden. Dies kann der Maximalwertbildung entsprechen. Vorteilhaft wird das defekte Detektorelement ausgeglichen bzw. korrigiert, wobei die Information über das Untersuchungsobjekt auf realen Messwerten basiert, im Gegensatz zu geschätzten Messwerten aus einem Interpolationsschritt.

Gemäß einem Aspekt der Erfindung wird die Maximalwertbildung für Messwerte der Aufnahmen ausgeführt, bei denen mindestens ein Messwert des Detektorelements in einer Aufnahme unter einem Schwellenwert liegt, und sonst wird die Mittelwertbildung ausgeführt. Defekte Detektorelemente können maskiert sein, wobei jedem defekten Detektorelement ein (Mess-)Wert gleich Null zugeordnet wird. Der Schwellwert kann derart gewählt werden, dass den defekten Detektorelementen der Maximalwert der anderen Aufnahme bzw. der anderen Aufnahmen zugeordnet wird. Alternativ kann eine Abfrage erfolgen, welchem Detektorelement der Messwert gleich Null zugeordnet ist und für diese defekten Detektorelemente wird die Maximalwertbildung angewendet. Vorteilhaft können die defekten Detektorelemente ausgeglichen werden.

Gemäß einem Aspekt der Erfindung wird an Positionen von defekten Detektorelementen einer Aufnahme nur die jeweils andere Aufnahme zur Kombination zu dem Bilddatensatz verwendet. Dies kann einer Maximalwertbildung entsprechen. Vorteilhaft können die Messwerte der defekten Detektorelemente vernachlässigt werden und dennoch ein vollständiger Bilddatensatz aufgenommen werden.

Gemäß einem Aspekt der Erfindung werden die Aufnahmen miteinander registriert. Die Aufnahmen werden bevorzugt miteinander registriert. Die Registrierung kann mit Hilfe von zumindest einem Marker erfolgen. Der Marker kann beispielsweise für Röntgenstrahlung hochabsorbierend sein, beispielsweise ein metallisches Material wie Wolfram oder Eisen aufweisen. Der Marker kann am Untersuchungsobjekt angeordnet bzw. am Untersuchungsobjekt temporär befestigt sein. Der Marker kann alternativ eine anatomische Struktur des Untersuchungsobjekts darstellen, beispielsweise aufweisend Knochen. Zum Beispiel kann die zweite Aufnahme bzw. können alle weiteren Aufnahmen auf der ersten Aufnahme als Referenz registriert werden. Durch die Registrierung werden gleiche Bildbereiche, welche die gleiche Objektregion abbilden, zwischen den jeweiligen Aufnahmen übereinstimmend angeordnet. Dadurch ergibt sich zugleich auch die örtliche bzw. lokale Zuordnung der jeweiligen Detektorelemente zu einem Bildpunkt. Vorteilhaft können Artefakte durch die Verschiebung vermieden werden.

Gemäß einem Aspekt der Erfindung ist ein Röhrenstrom der Röntgenquelle jeweils für die erste Aufnahme und die zweite Aufnahme im Wesentlichen gleich. Vorteilhaft können beispielsweise Mittelwertbildung oder Maximalwertbildung ohne eine (Intensitäts-)Korrektur zum Ausgleich unterschiedlicher Röhrenströme bei der ersten und zweiten Aufnahme angewendet werden. Idealerweise ist die Summe aus der jeweiligen Dosis der ersten Aufnahme und der zweiten Aufnahme, und ggf. weiterer Aufnahmen, im Wesentlichen gleich der Dosis einer einzigen normalen Aufnahme eines Bilddatensatzes, welche keine Kombination von einer ersten Aufnahme und einer zweiten Aufnahme aufweist. Im Gegensatz zu einer einzigen normalen Aufnahme kann der Röhrenstrom für die erste Aufnahme und die zweite Aufnahme beispielsweise proportional zur Anzahl der Aufnahmen bzw. Positionen gewählt werden. Vorteilhaft kann die Bildqualität bei gleichbleibender Dosis verbessert werden. Vorteilhaft kann für die funktionsfähigen Detektorelemente eine gleichbleibende Bildqualität erreicht werden, während die Bildqualität für die defekten Detektorelemente verbessert wird.

Gemäß einem Aspekt der Erfindung wird im Schritt des Verschiebens zusätzlich die spektrale Zusammensetzung der Röntgenstrahlung verändert. Das Verändern der spektralen Zusammensetzung der Röntgenstrahlung kann beispielsweise mittels Umschalten zwischen Röhrenspannungen oder mittels Springen zwischen Anodenbahnen ausgebildet sein. Beispielsweise kann eine Anodenbahn Molybdän und die andere Anodenbahn Wolfram aufweisen. Das Verändern der spektralen Zusammensetzung der Röntgenstrahlung kann insbesondere für mammographische Anwendungen vorteilhaft sein. Vorteilhaft kann zumindest im Bereich der funktionsfähigen Detektorelemente zusätzlich spektrale bzw. materialspezifische Zusatzinformation über die Untersuchungsregion bzw. Objektregion gewonnen werden. In den Bereichen defekter Detektorelement bzw. bei Verwendung der Maximalwertbildung kann die spektrale Information aus benachbarten funktionsfähigen Detektorelemente interpoliert werden.

Gemäß einem Aspekt der Erfindung bewegen sich im Schritt des Verschiebens zusätzlich die Röntgenquelle oder/und der Röntgendetektor relativ zum zu untersuchenden Objekt. Vorteilhaft können tomographische oder tomosynthetische Bilddatensätze erzeugt werden. Beispielsweise können die Röntgenquelle oder/und der Röntgendetektor sich auf einer Kreisbahn oder entlang einer linearen Strecke bewegen. Bevorzugt kann der Röntgenquellen-Röntgendetektor-Abstand während der Aufnahme bzw. für die einzelnen Aufnahmen konstant gewählt werden. Bei unterschiedlichem Röntgenquellen-Röntgendetektor-Abstand bei der ersten Aufnahme und der zweiten Aufnahme oder bei etwaiger Verzerrung eines C-Bogen-Systems kann eine zusätzliche Korrektur des Röntgenquellen-Röntgendetektor-Abstand vor der Kombination der ersten Aufnahme und der zweiten Aufnahme und ggf. weiterer Aufnahmen erfolgen, so dass die Objektregion korrekt im Bilddatensatz dargestellt werden kann.

Die Aufnahme des Bilddatensatz umfasst radiologische, vorzugsweise mammographische, Aufnahmen. Bevorzugt sind die Aufnahmen radiologische, besonders bevorzugt mammographische, Aufnahmen. Die erste Aufnahme und die zweite Aufnahme sowie ggf. die weiteren Aufnahmen sind also bevorzugt Röntgenaufnahmen, die besonders bevorzugt als Projektionsbild von der Brust eines menschlichen Untersuchungsobjekts erfasst werden.

Die Erfindung betrifft ferner eine Röntgenquelle ausgelegt zum Durchführen eines erfindungsgemäßen Verfahrens aufweisend eine Verschiebeeinheit zum Verschieben des Brennflecks um den Abstand und eine Steuereinheit zum Steuern des Verschiebens des Brennflecks. Die Verschiebeeinheit kann eine elektrostatische oder magnetische Ablenkungseinheit des Elektronenstrahls in der Röntgenquelle umfassen, so dass der Auftreffpunkt des Elektronenstrahls auf der Anode verändert wird. Alternativ kann die Verschiebeeinheit mittels zweier Emitter jeweils in Kombination mit einem, insbesondere schaltbaren, Gitter oder mittels zweier Emitter in Form von CNT (engl.: carbon nano tube) bzw. Kaltemitter ausgebildet sein, wodurch der Elektronenstrahl jeweils nur von einem Emitter ausgesendet wird. Die Steuereinheit kann eine Steuerung des Abstands ermöglichen. Die Steuereinheit kann mittels eines Signals, welches die Information über den Abstand aufweist, oder dem Abruf eines gespeicherten Abstands den Abstand steuern bzw. einstellen. Besonders vorteilhaft weist die Steuereinheit eine Verbindung zum Röntgendetektor auf, wodurch beispielsweise die Information über den Abstand übertragen werden kann.

Die Erfindung betrifft ferner ein medizinisches Gerät aufweisend Röntgendetektor, eine erfindungsgemäße Röntgenquelle und eine Rechnereinheit. Die Rechnereinheit kann insbesondere zur Kombination der ersten und der zweiten Aufnahme ausgebildet sein. Die Rechnereinheit kann zum Bestimmen der Clustergröße oder des Abstands ausgebildet sein. Die Rechnereinheit kann die Messwerte des Röntgendetektors der ersten Aufnahme und der zweiten Aufnahme sowie die Parameter der Röntgenquelle, beispielsweise die erste oder zweite Position des Brennpunkts, empfangen und kombinieren. Die Rechnereinheit kann zusätzliche Schritte der Korrektur vor, nach oder während der Kombination der ersten Aufnahme und der zweiten Aufnahme durchführen. Die Rechnereinheit kann den Bilddatensatz bereitstellen. Die Rechnereinheit kann ferner weitere Schritte der Rekonstruktion basierend auf dem Bilddatensatz bzw. einer Mehrzahl von Bilddatensätzen durchführen.

Die Erfindung betrifft ferner eine Verwendung eines erfindungsgemäßen medizinischen Geräts zur medizinischen Bildgebung, wobei zumindest zwei Aufnahmen erfasst werden, zwischen denen der Brennfleck um einen Abstand verschoben wird, und aus den zumindest zwei Aufnahmen ein kombinierter Bilddatensatz ermittelt wird. Das medizinische Gerät kann zu einer gezielten Steuerung des Brennflecks basierend auf der Anordnung der defekten Detektorelemente innerhalb der Detektionsfläche ausgelegt sein.

Die Erfindung betrifft ferner ein Computerprogrammprodukt mit einem Computerprogramm, welches direkt in eine Speichereinrichtung einer Steuereinrichtung eines medizinischen Geräts ladbar ist, mit Programmabschnitten, um alle Schritte eines erfindungsgemäßen Verfahrens auszuführen, wenn das Computerprogramm in der Steuereinrichtung des medizinischen Geräts ausgeführt wird. Ein solches Computerprogrammprodukt kann neben dem Computerprogramm gegebenenfalls zusätzliche Bestandteile wie z. B. eine Dokumentation und/oder zusätzliche Komponenten auch Hardware-Komponenten, wie z.B. Hardware-Schlüssel (Dongles etc.) zur Nutzung der Software, umfassen.

Die wesentlichen Komponenten zur Steuerung der Röntgenquelle und des Röntgendetektors können zum überwiegenden Teil in Form von Softwarekomponenten ausgebildet sein. Grundsätzlich können diese Komponenten aber auch zum Teil, insbesondere wenn es um besonders schnelle Berechnungen geht, in Form von softwareunterstützter Hardware, beispielsweise FPGAs oder dergleichen, realisiert sein. Ebenso können die benötigten Schnittstellen, beispielsweise wenn es nur um eine Übernahme von Daten aus anderen Softwarekomponenten geht, als Softwareschnittstellen ausgebildet sein. Sie können aber auch als hardwaremäßig aufgebaute Schnittstellen ausgebildet sein, die durch geeignete Software angesteuert werden.

Die Erfindung betrifft ferner ein computerlesbares Medium, auf welchem von einer Rechnereinheit einlesbare und ausführbare Programmabschnitte gespeichert sind, um alle Schritte eines erfindungsgemäßen Verfahrens auszuführen, wenn die Programmabschnitte von der Rechnereinheit ausgeführt werden. Zum Transport zur Steuerungseinrichtung und/oder zur Speicherung an oder in der Steuerungseinrichtung kann ein computerlesbares Medium, beispielsweise ein Memorystick, eine Festplatte oder ein sonstiger transportabler oder fest eingebauter Datenträger dienen, auf welchem die von einer Rechnereinheit der Steuerungseinrichtung einlesbaren und ausführbaren Programmabschnitte des Computerprogramms gespeichert sind. Die Rechnereinheit kann z.B. hierzu einen oder mehrere zusammenarbeitende Mikroprozessoren oder dergleichen aufweisen.

Nachfolgend werden Ausführungsbeispiele der Erfindung anhand von Zeichnungen näher erläutert. Hierbei zeigt:
FIG 1 eine schematische Darstellung des erfindungsgemäßen Verfahrens in einer ersten Ausführungsform;
FIG 2 eine schematische Darstellung des erfindungsgemäßen medizinischen Geräts in einer ersten Ausführungsform;
FIG 3 eine schematische Darstellung einer idealen Aufnahme eines Bilddatensatzes;
FIG 4 eine schematische Darstellung des erfindungsgemäßen Verfahrens in einer zweiten Ausführungsform;
FIG 5 eine schematische Darstellung des erfindungsgemäßen Verfahrens in einer dritten Ausführungsform; und
FIG 6 eine schematische Darstellung des erfindungsgemäßen medizinischen Geräts in einer zweiten Ausführungsform;

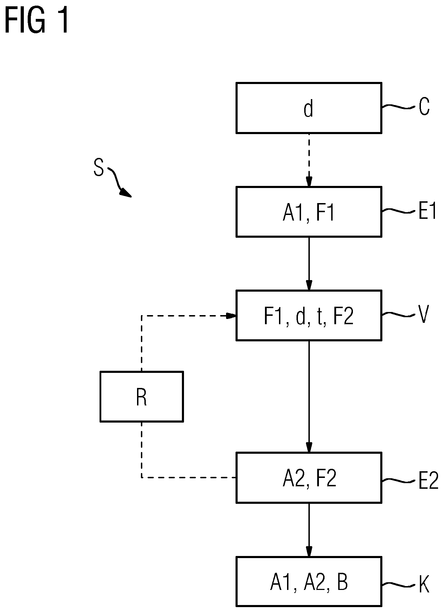

Die Fig. 1 zeigt eine beispielhafte Ausführung des erfindungsgemäßen Verfahrens S in einer ersten Ausführungsform. Das Verfahren S zur Aufnahme von einem Bilddatensatz B wird mit einem Röntgendetektor umfassend eine Detektionsfläche mit einer Vielzahl von Detektorelementen und einer Röntgenquelle mit einem Brennfleck durchgeführt. Das Verfahren S umfasst die Schritte, bevorzugt in der folgenden Reihenfolge, des etwaigen Bestimmens C des Abstands d, des erstens Erfassens E1, des Verschiebens V, des zweiten Erfassens E2, der etwaigen Wiederholung R der Schritte des Verschiebens V und des zweiten Erfassens E2, und der Kombination K. Im Schritt des ersten Erfassens wird eine erste Aufnahme A1 mit einer ersten Position F1 des Brennflecks erfasst bzw. aufgenommen. Im Schritt des Verschiebens V wird der Brennfleck von der ersten Position F1 zu einer von der ersten Position F1 verschiedenen zweiten Position F2 des Brennflecks um einen Abstand d verschoben. Das Verschieben weist eine Verschiebezeit t auf. Im Schritt des zweiten Erfassens E2 wird eine zweite Aufnahme A2 mit der zweiten Position F2 erfasst bzw. aufgenommen. Im Schritt der Kombination K werden die erste Aufnahme A1 und die zweite Aufnahme A2 zum Bilddatensatz B kombiniert.

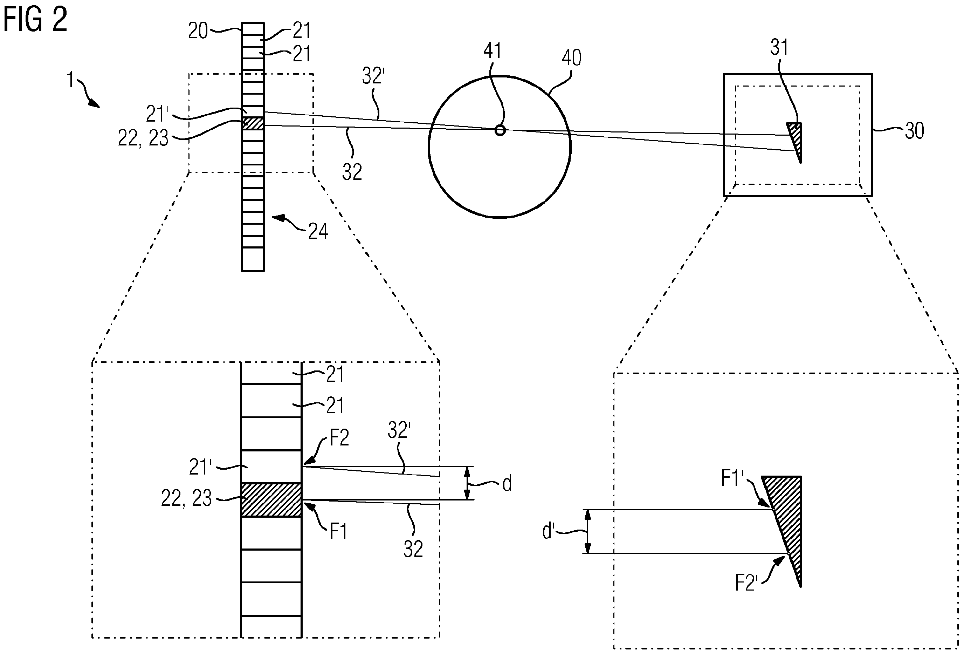

Die Fig. 2 zeigt eine beispielhafte Ausführung des erfindungsgemäßen medizinischen Geräts 1 in einer ersten Ausführungsform. Das medizinische Gerät 1 ist insbesondere für radiographisch oder mammographische Aufnahmen geeignet. Das medizinische Gerät weist einen Röntgendetektor 20 und ein Röntgenquelle 30 auf. Der Röntgendetektor 20 ist vorzugsweise ein Röntgen-Flachdetektor. Der Röntgendetektor 20 umfasst funktionsfähige Detektorelemente 21 und zumindest ein defektes Detektorelement 22 bzw. eine Lücke 23 zwischen benachbarten Detektorsegmenten aufweisend eine Mehrzahl von funktionsfähigen Detektorelementen 21 und ggf. defekten Detektorelementen 22. Der Röntgendetektor 20 weist eine Detektionsfläche 24 auf, welche durch eine Oberfläche der funktionsfähigen Detektorelemente 21 und ggf. den defekten Detektorelementen 22 gebildet wird. Die Detektionsfläche 24 ist im Wesentlich senkrecht zu einem Zentralstrahl der Röntgenquelle 30 ausgerichtet. Die Röntgenquelle 30 umfasst eine Anode 31, welche als Anodenteller ausgebildet sein kann. Das Untersuchungsobjekt 40 ist zwischen dem Röntgendetektor 20 und der Röntgenquelle 30 angeordnet, so dass die Objektregion 41 in der ersten Aufnahme und der zweiten Aufnahme erfasst wird.

Im vergrößert dargestellten Bereich des Röntgendetektors 20 trifft der Zentralstrahl 32 der Röntgenquelle 30 im Wesentlichen senkrecht auf das defekte Detektorelement 22 bzw. die Lücke 23. Dabei ist die erste Position F1 am Schnittpunkt der Oberfläche des defekten Detektorelements 22 und des Zentralstrahls 32 angeordnet. Im vergrößert dargestellten Bereich der Röntgenquelle 30 ist die erste Position F1' auf der Oberfläche der Anode dargestellt. Nach dem Verschieben trifft der Zentralstrahl 32' der Röntgenquelle 30 auf ein dem defekten Detektorelement 22 bzw. der Lücke 23 benachbartes funktionsfähiges Detektorelement 21'. Dabei ist die zweite Position F2 am Schnittpunkt der Oberfläche des benachbarten funktionsfähigen Detektorelements 21' und des Zentralstrahls 30' angeordnet. Die zweite Position F2 ist um den Abstand d gegenüber der ersten Position F1 verschoben. Die zweite Position F2' auf der Oberfläche der Anode ist um den Abstand d' gegenüber der ersten Position F1' verschoben. Der Zentralstrahl 32 und der Zentralstrahl 32' passieren die Objektregion 41. Damit kann das benachbarte funktionsfähige Detektorelement 21 und das defekte Detektorelement 22 bzw. die Lücke 23 über die Zentralstrahlen 32, 32' räumlich zueinander zugeordnet werden. Der Abstand d beträgt hier beispielsweise n=1, d.h. der Abstand zwischen der ersten Position F1 und der zweiten Position F2 beträgt ein Detektorelement bzw. eine Einheit der Erstreckung der Detektorelemente 21,21',22. Die Messwerte des defekten Detektorelements 22 und des benachbarten funktionsfähigen Detektorelements 21' werden kombiniert. Alternativ wird der Messwert des benachbarten funktionsfähigen Detektorelements 21' im Schritt des Kombinierens als Messwert für die Objektregion 41 verwendet.

Das heißt, es wird eine Transformation zwischen den Aufnahmen A1, A2 erzeugt, die im Wesentlichen das Verschieben V ausgleicht und in beiden Aufnahmen A1, A2 gleiche Strukturen einander räumlich zuordnet. Diese räumliche Zuordnung ist hier beispielhaft durch die Trajektorie von der Röntgenquelle 30 emittierten Zentralstrahlen 32, 32' veranschaulicht. Es werden also die Detektorelemente 21, 22 einander zugeordnet, die in demselben Strahlengang der Zentralstrahlen bzw. entsprechend anderen Röntgenstrahlen des Strahlenbündels angeordnet sind.

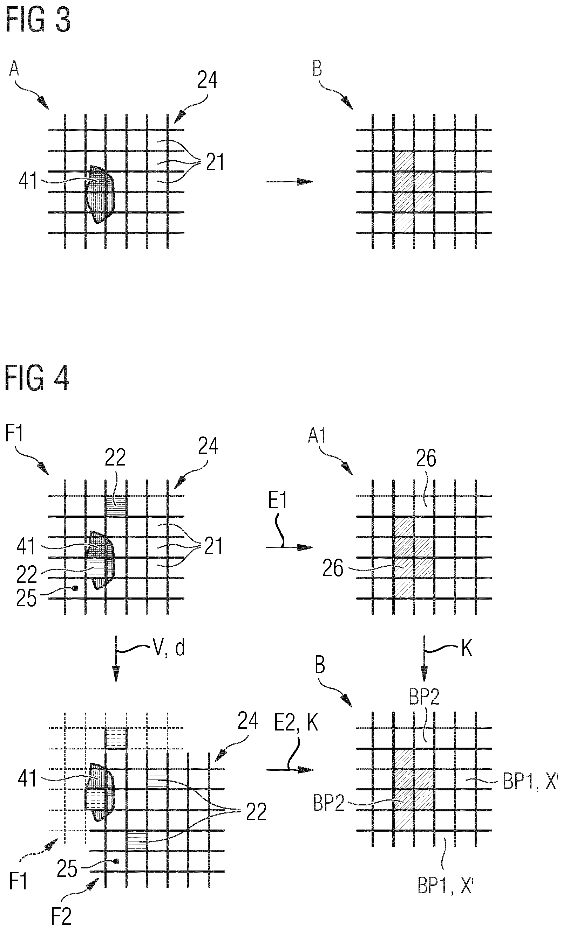

In Fig. 3 ist schematisch eine idealisierte Aufnahme eines Bilddatensatzes B dargestellt. Ein idealer Strahlungsdetektor A weist eine Detektionsfläche 24 auf, in der alle Detektorelemente 21 funktionsfähig sind. Auf die Detektionsfläche 24 wird eine Projektion einer interessierenden Objektregion 41 mit Hilfe von Röntgenstrahlung abgebildet und von den einzelnen Detektorelementen 21 detektiert. Aus den Detektorelementen 21 werden Messwerte ausgelesen, die in diesem Idealfall auch gleich den Bildpunktwerten entsprechen, da alle Detektorelemente optimal funktionieren. Die die einzelnen Detektorelemente und ihre Messwerte umfassende Aufnahme entspricht in diesem Fall auch gleich einem optimal akquirierten, idealen Bilddatensatz B, welcher die interessierende Objektregion 41 als Bildpunkte mit zugeordneten Bildpunktwerten (hier durch unterschiedlich starke Schraffuren kenntlich gemacht) darstellt.

Die Fig. 4 zeigt eine beispielhafte Ausführung des erfindungsgemäßen Verfahrens S in einer zweiten Ausführungsform. Die zunächst mit der ersten Position F1 beleuchtete Detektionsfläche 24 eines Ausführungsbeispiels eines Röntgendetektors 20 ist ähnlich zu der Detektionsfläche 24 des anhand von Fig. 3 beschriebenen idealen Strahlungsdetektors A. Im Gegensatz dazu weist sie jedoch zwei defekte Detektorelemente 22 auf, wobei eines der defekten Detektorelemente 22 im Bereich der interessierenden Objektregion 41 angeordnet ist. Zudem weist die Detektionsfläche 24 in einem Pixel 21 einen Marker 25, z. B. einen starken Röntgenabsorber, auf, mit dessen Hilfe ein Verschieben V möglichst exakt bestimmt werden kann.

Mit der ersten Position F1 wird eine Aufnahme A1 erfasst, in der die interessierende Objektregion 41 aufgrund der defekten Detektorelemente 22 jedoch nicht korrekt abgebildet werden kann. Denn es werden an Positionen 26 in der Aufnahme A1 Messwerte ausgelesen, die nicht die tatsächliche Struktur in der interessierenden Objektregion 41 repräsentieren.

Die Detektionsfläche 24 ist zudem unter Beleuchtung mit der zweiten Position F2 dargestellt. Darin ist sie im Verhältnis zu der interessierenden Objektregion 41 um einen Abstand d verschoben, also um einen diagonalen Verschiebevektor, um zwei Pixel verschoben. Die Detektionsfläche 24 ist hier zusätzlich (zur Veranschaulichung das Verschieben V um den Abstand d) schraffiert unter Beleuchtung mit der ersten Position F1 dargestellt. Mit der Detektionsfläche 24 wird auch der Marker 26 verschoben, sodass über einen Abgleich der Position des Markers 26 das Verschieben um den Abstand d ermittelt werden kann und die einzelnen Aufnahmen A1 und A2 miteinander registriert werden können.

Mit der zweiten Position F2 wird eine zweite Aufnahme im Schritt des zweiten Erfassens E2 erfasst und die zweite Aufnahme wird mit der ersten Aufnahme A1 im Schritt des Kombinierens K kombiniert. Daraus resultiert der Bilddatensatz B, in dem an den Bildpunkten BP2, an denen in der ersten Aufnahme A1 (an den Positionen 26) keine korrekten Messwerte ermittelt werden konnten, die Messwerte der zweiten Aufnahme als Bildpunktwerte verwendet werden. An den Bildpunkten BP1, an deren Positionen 26' in der zweiten Aufnahme A2 die defekten Detektorelemente angeordnet waren, werden die Messwerte der ersten Aufnahme A1 als Bildpunktwerte verwendet. An allen übrigen Bildpunkten können die Bildpunktwerte mittels einer Mittelwertbildung erhalten werden. Das mittels des erfindungsgemäßen Verfahrens erhaltene Bilddatensatz B entspricht dabei trotz der defekten Detektorelemente 22 des realen Röntgendetektors im Wesentlichen dem idealen Bilddatensatz B aus FIG 3.

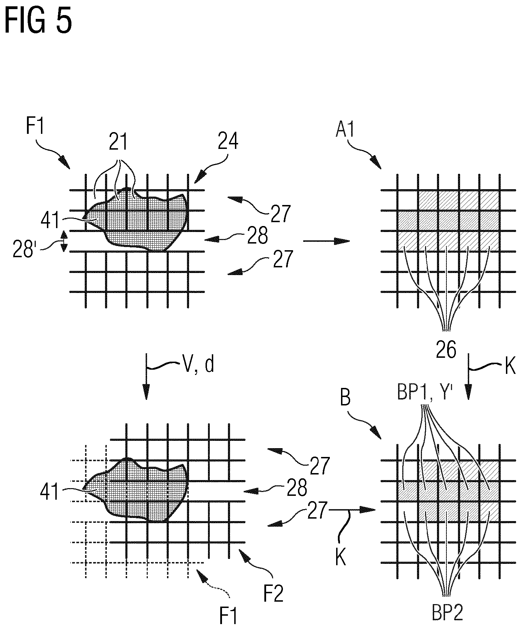

Die Fig. 5 zeigt eine beispielhafte Ausführung des erfindungsgemäßen Verfahrens S in einer dritten Ausführungsform. Der Röntgendetektor 20 weist eine Detektionsfläche 24 mit Detektorsegmenten 27 und dazwischen angeordneten Fugen 28 auf. Zwischen den Detektorsegmenten 27 erstreckt sich die Fuge 28 mit einer Fugenbreite 28' von beispielsweise 400 µm (also 5-10 Pixeln, hier nicht maßstabsgetreu dargestellt). Die erste Aufnahme A1 wird mit der ersten Position F1 erfasst, wobei an Positionen 26, in denen sich die Fuge 28 befindet, keine korrekten Pixelwerte ermittelt werden können. Der Brennfleck wird für eine zweite Aufnahme A2 in die zweite Position F2 verschoben, hier jedoch um zumindest eine Fugenbreite 28' mit einen schrägen Verschiebevektor um den Abstand d. Durch das Verschieben V sind mit der zweiten Position F2 funktionsfähige Detektorelemente 21 in einem Bereich, in dem mit der ersten Position F1 die Fuge 28 angeordnet war. Dadurch kann mit Hilfe einer Kombination der beiden Aufnahmen A1, A2 der Bilddatensatz B erzeugt werden.

An den Bildpunkten BP2, an denen in der ersten Aufnahme A1 (an den Positionen 26) keine korrekten Messwerte ermittelt werden konnten, können die Messwerte der zweiten Aufnahme als Bildpunktwerte verwendet werden. An den Bildpunkten BP1, an deren Positionen 26' in der zweiten Aufnahme die defekten Detektorelemente 22 angeordnet waren, werden die Messwerte der ersten Aufnahme A1 als Bildpunktwerte verwendet. Auch hier entspricht der mittels des erfindungsgemäßen Verfahrens erhaltene Bilddatensatz B trotz der Fuge 28 zwischen den Detektorsegmenten 27 des realen Röntgendetektors 20 im Wesentlichen dem idealen Bilddatensatz B.

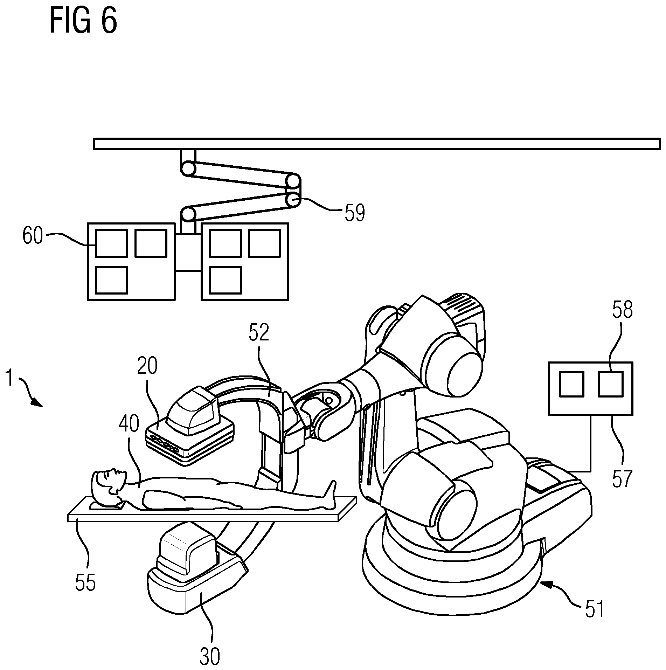

Die Fig. 6 zeigt eine beispielhafte Ausführung eines erfindungsgemäßen medizinischen Geräts 1 in einer zweiten Ausführungsform, welche als C-Bogen-System zur Schichtbildgebung ausgebildet ist. Das medizinische Gerät 1 weist einen Ständer 51, beispielsweise in Form eines Industrieroboters, mit einem vom Ständer 51 gehaltenen C-Bogen 52 auf. An den Enden des C-Bogens 52 sind eine Röntgenquelle 30 sowie der Röntgendetektor 20 angebracht. Der C-Bogen 52 kann mittels der durch den Ständer 51 bereitgestellten Freiheitsgrade positioniert werden. Im Strahlengang der Röntgenquelle 30 befindet sich auf einer Tischplatte 55 eines Patientenlagerungstisches ein zu untersuchendes Untersuchungsobjekt 40. Am medizinischen Gerät 1 ist eine Systemsteuerungseinheit 57 mit einem Hochspannungsgenerator zur Erzeugung der Röhrenspannung und einem Recheneinheit 58 angeschlossen. Die Recheneinheit 58 empfängt und verarbeitet die Bildwerte der ersten und zweiten Aufnahme und für den Schritt der Kombination durch. Der Bilddatensatz kann dann auf Displays einer mittels eines deckenmontierten Trägersystems 59 gehaltenen Monitorampel 60 betrachtet werden. Das Trägersystem 59 weist dabei Verfahr-, Schwenk- Dreh- und/oder Höhenverstelleinheiten auf. In der Systemsteuerungseinheit 57 ist weiterhin eine Verarbeitungsschaltung 61 vorgesehen.

Obwohl die Erfindung im Detail durch das bevorzugte Ausführungsbeispiel näher illustriert wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren (S) zur Aufnahme von einem Bilddatensatz (B) mit einem Röntgendetektor (20) umfassend eine Detektionsfläche (24) mit einer Vielzahl von Detektorelementen (21, 22) und einer Röntgenquelle (30) mit einem Brennfleck, umfassend die folgenden Schritte:
a. Erstes Erfassen (E1) von einer ersten Aufnahme (A1) mit einer ersten Position (F1) des Brennflecks,
b. Verschieben (V) des Brennflecks von der ersten Position (F1) zu einer von der ersten Position verschiedenen zweiten Position (F2) des Brennflecks um einen Abstand (d),
c. Zweites Erfassen (E2) einer zweiten Aufnahme (A2) mit der zweiten Position (F2),
d. Kombination (K) der ersten Aufnahme (A1) und der zweiten Aufnahme (A2) zum Bilddatensatz (B),
**dadurch gekennzeichnet, dass** der Röntgendetektor (20) in Detektorsegmente (27) mit jeweils einer Anzahl von Detektorelementen (21, 22) unterteilt ist und zwischen den Detektorsegmenten (27) Fugen (28) mit einer Fugenbreite (28') angeordnet sind, und der Abstand (d) in der Projektion auf die Detektionsfläche (24) um zumindest eine Fugenbreite (28') verschoben wird.

2. Verfahren (S) nach Anspruch 1, wobei der Abstand (d) in der Projektion auf die Detektionsfläche (24) um n oder n+0,5 Detektorelemente (21, 22) beträgt, wobei n eine natürliche Zahl ist.

3. Verfahren (S) nach einem der vorangehenden Ansprüche, wobei der Abstand (d) basierend auf einer Clustergröße von defekten Detektorelementen (22) bestimmt wird.

4. Verfahren (S) nach einem der vorangehenden Ansprüche, wobei eine Verschiebezeit (t) des Brennflecks kürzer oder gleich einer Auslesezeit des Röntgendetektors (20) ist.

5. Verfahren (S) nach einem der vorangehenden Ansprüche, wobei die Kombination (K) der Aufnahmen (A1, A2) eine pixelweise Mittelwertbildung und/oder eine pixelweise Maximalwertbildung umfasst.

6. Verfahren (S) nach Anspruch 5, wobei die Maximalwertbildung für Messwerte der Aufnahmen (A1, A2) ausgeführt wird, bei denen mindestens ein Messwert des Detektorelements (22) in einer Aufnahme (A1, A2) unter einem Schwellenwert liegt, und sonst die Mittelwertbildung ausgeführt wird.

7. Verfahren (S) nach einem der vorangehenden Ansprüche, wobei an Positionen von defekten Detektorelementen (22) einer Aufnahme (A1) nur die jeweils andere Aufnahme (A2) zur Kombination (K) zu dem Bilddatensatz (B) verwendet wird.

8. Verfahren (S) nach einem der vorangehenden Ansprüche, wobei die Aufnahmen (A1, A2) miteinander registriert werden.

9. Verfahren (S) nach einem der vorangehenden Ansprüche, wobei ein Röhrenstrom der Röntgenquelle (30) jeweils für die erste Aufnahme (A1) und die zweite Aufnahme (A2) im Wesentlichen gleich ist.

10. Verfahren (S) nach einem der vorangehenden Ansprüche, wobei im Schritt des Verschiebens (V) zusätzlich die spektrale Zusammensetzung der Röntgenstrahlung verändert wird.

11. Verfahren (S) nach einem der vorangehenden Ansprüche, wobei sich im Schritt des Verschiebens (V) zusätzlich die Röntgenquelle (30) oder/und der Röntgendetektor (20) relativ zum zu untersuchenden Objekt (40) bewegen.

12. Röntgenquelle (30) ausgelegt zum Durchführen eines Verfahrens (S) nach einem der vorangehenden Ansprüche aufweisend eine Verschiebeeinheit zum Verschieben (V) des Brennflecks um den Abstand (d) und eine Steuereinheit zum Steuern des Verschiebens (V) des Brennflecks.

13. Medizinisches Gerät (1) aufweisend Röntgendetektor (20), Röntgenquelle (30) nach Anspruch 12 und eine Rechnereinheit.

14. Verwendung eines medizinischen Geräts (1) nach Anspruch 13 zur medizinischen Bildgebung, wobei zumindest zwei Aufnahmen (A1, A2) erfasst werden, zwischen denen der Brennfleck um einen Abstand (d) verschoben wird, und aus den Aufnahmen (A1, A2) ein kombinierter Bilddatensatz (B) ermittelt wird.

15. Computerprogrammprodukt mit einem Computerprogramm, welches direkt in eine Speichereinrichtung einer Steuereinrichtung eines medizinischen Geräts nach Anspruch 13 ladbar ist, mit Programmabschnitten, um alle Schritte eines Verfahrens (S) nach einem der Ansprüche 1 bis 11 auszuführen, wenn das Computerprogramm in der Steuereinrichtung des medizinischen Geräts nach Anspruch 13 (1) ausgeführt wird.

16. Computerlesbares Medium, auf welchem von einer Rechnereinheit eines medizinischen Geräts nach Anspruch 13 einlesbare und ausführbare Programmabschnitte gespeichert sind, um alle Schritte eines Verfahrens nach einem der Ansprüche 1 bis 11 auszuführen, wenn die Programmabschnitte von der Rechnereinheit des medizinischen Geräts nach Anspruch 13 ausgeführt werden.

## Claims

1. Method (S) for recording an image data set (B) with an x-ray detector (20) comprising a detection surface (24) with a plurality of detector elements (21, 22) and an x-ray source (30) with a focal point, comprising the following steps:
a. first capturing (E1) a first recording (A1) with a first position (F1) of the focal point,
b. moving (V) the focal point from the first position (F1) to a second position (F2) of the focal point, which differs from the first position, by a distance (d),
c. second capturing (E2) a second recording (A2) with the second position (F2),
d. combining (K) the first recording (A1) and the second recording (A2) to form the image data set (B),
**characterised in that** the x-ray detector (20) is divided into detector segments (27) with in each case a number of detector elements (21, 22) and joints (28) with a joint width (28') are arranged between the detector segments (27), and the distance (d) in the projection onto the detection surface (24) is moved by at least one joint width (28').

2. Method (S) according to claim 1, wherein the distance (d) in the projection onto the detection surface (24) amounts to n or n+0.5 detector elements (21, 22), wherein n is a natural number.

3. Method (S) according to one of the preceding claims, wherein the distance (d) is determined on the basis of a cluster size of faulty detector elements (22).

4. Method (S) according to one of the preceding claims, wherein a displacement time (t) of the focal point is shorter or equal to a read-out time of the x-ray detector (20).

5. Method (S) according to one of the preceding claims, wherein the combination (K) of the recordings (A1, A2) comprises a mean value and/or a maximum value formation pixel by pixel.

6. Method (S) according to claim 5, wherein the maximum value formation for measured values of the recordings (A1, A2) is executed, in which at least one measured value of the detector element (22) in a recording (A1, A2) lies below a threshold value, and otherwise the mean value formation is executed.

7. Method (S) according to one of the preceding claims, wherein at positions of faulty detector elements (22) of a recording (A1) only the respective other recording (A2) is used for combination purposes (K) to form the image data set (B) .

8. Method (S) according to one of the preceding claims, wherein the recordings (A1, A2) are registered with one another.

9. Method (S) according to one of the preceding claims, wherein a tube current of the x-ray source (30) is essentially identical in each case for the first recording (A1) and the second recording (A2).

10. Method (S) according to one of the preceding claims, wherein in the movement step (V) the spectral composition of the x-ray radiation is additionally changed.

11. Method (S) according to one of the preceding claims, wherein in the step of moving (V) the x-ray source (30) or/and the x-ray detector (20) additionally move relative to the object under examination (40).

12. X-ray source (30) designed to perform a method (S) according to one of the preceding claims, having a movement unit for moving (V) the focal point by the distance (d) and a control unit for controlling the movement (V) of the focal point.

13. Medical device (1) having an x-ray detector (20), x-ray source (30) according to claim 12 and a computer unit.

14. Use of a medical device (1) according to claim 13 for medical imaging, wherein at least two recordings (A1, A2) are captured, between which the focal point is moved by a distance (d), and a combined image data set (B) is determined from the recordings (A1, A2).

15. Computer program product with a computer program, which can be loaded directly into a storage facility of a control facility of a medical device according to claim 13, having program sections for executing all steps of a method (S) according to one of claims 1 to 11, if the computer program is executed in the control facility of the medical device according to claim 13.

16. Computer-readable medium, on which program sections which can be read in and executed by a computer unit of a medical device according to claim 13 are stored, in order to carry out all steps of a method according to one of claims 1 to 11, if the program sections are executed by the computer unit of the medical device according to claim 13.

## Revendications

1. Procédé (S) d'enregistrement d'un ensemble (B) de données d'image par un détecteur (20) de rayons X comprenant une surface (24) de détection ayant une pluralité d'éléments (21, 22) de détecteur et une source (30) de rayonnement ayant une tâche focale, comprenant les stades suivants :
a. première saisie (E1) d'un premier enregistrement (A1) à une première position (F1) de la tâche focale,
b. déplacement (V) de la tâche focale de la première position (F1) à une deuxième position (F2) de la tâche focale différente de la première position d'une distance (d),
c. deuxième saisie (E2) d'un deuxième enregistrement (A2) à la deuxième position (F2),
d. combinaison (K) du premier enregistrement (A1) et du deuxième enregistrement (A2) en l'ensemble (B) de données d'image,
**caractérisé en ce que** le détecteur (20) de rayons X est subdivisé en segments (27) de détecteur ayant chacun un certain nombre d'éléments (21, 22) de détecteur et des joints (28) ayant une largeur (28') de joint sont disposés entre les segments (27) du détecteur et on déplace, d'au moins une largeur (28') de joint, la distance (d) dans la projection sur la surface (24) de détection.

2. Procédé (S) suivant la revendication 1, dans lequel la distance (d) dans la projection sur la surface (24) de détection représente n ou n+0,5 éléments (21, 22) de détecteur, n étant un nombre naturel.

3. Procédé (S) suivant l'une des revendications précédentes, dans lequel on détermine la distance (d) sur la base d'une grandeur de groupe d'éléments (22) de détecteur défectueux.

4. Procédé (S) suivant l'une des revendications précédentes, dans lequel un temps (t) de déplacement de la tâche focale est inférieur ou égal à un temps de lecture du détecteur (20) de rayons X.

5. Procédé (S) suivant l'une des revendications précédentes, dans lequel la combinaison (K) des enregistrements (A1, A2) comprend une formation de valeur moyenne pixel par pixel et/ou une formation de valeur maximum pixel par pixel.

6. Procédé (S) suivant la revendication 5, dans lequel on effectue la formation de la valeur maximum pour des valeurs de mesure des enregistrements (A1, A2) pour lesquels au moins une valeur de mesure des éléments (22) de détecteur dans un enregistrement (A1, A2) est inférieure à une valeur de seuil, et sinon on effectue la formation de la valeur moyenne.

7. Procédé (S) suivant l'une des revendications précédentes, dans lequel on utilise, en des positions d'éléments (22) de détecteur défectueux d'un enregistrement (A1), seulement l'autre enregistrement (A2) respectif pour la combinaison (K) en l'ensemble (B) de données d'image.

8. Procédé (S) suivant l'une des revendications précédentes, dans lequel on enregistre l'un avec l'autre les enregistrements (A1, A2).

9. Procédé (S) suivant l'une des revendications précédentes, dans lequel un courant de rayons X de la source (30) de rayons X est sensiblement le même pour le premier enregistrement (A1) et le deuxième enregistrement (A2).

10. Procédé (S) suivant l'une des revendications précédentes, dans lequel, dans le stade du déplacement (V), on modifie en outre la composition spectrale du rayonnement X.

11. Procédé (S) suivant l'une des revendications précédentes, dans lequel, dans le stade du déplacement (V), on déplace en outre la source (30) de rayons X ou/et le détecteur (20) de rayons X par rapport à l'objet (40) à examiner.

12. Source (30) de rayons X conçue pour effectuer un procédé (S) suivant l'une des revendications précédentes, comportant une unité de déplacement pour le déplacement (V) de la tâche focale de la distance (d) et une unité de commande pour commander le déplacement (V) de la tâche focale.

13. Appareil (1) médical comportant un détecteur (20) de rayons X, une source (30) de rayons X suivant la revendication 12 et une unité informatique.

14. Utilisation d'un appareil (1) médical suivant la revendication 13 pour l'imagerie médicale, dans lequel on saisit au moins deux enregistrements (A1, A2), entre lesquels on déplace la tâche focale de la distance (d), et on détermine un ensemble (B) de données d'image combiné à partir des enregistrements (A1, A2).

15. Produit de programme d'ordinateur ayant un programme d'ordinateur, qui peut être chargé directement dans un dispositif de mémoire d'un dispositif de commande d'un appareil médical suivant la revendication 13, comprenant des parties de programme pour effectuer tous les stades d'un procédé (S) suivant l'une des revendications 1 à 11, lorsque le programme d'ordinateur est réalisé dans le dispositif de commande de l'appareil (1) médical suivant la revendication 13.

16. Support, déchiffrable par ordinateur, sur lequel sont mises en mémoire des parties de programme pouvant être déchiffrées et réalisées par une unité informatique d'un appareil médical suivant la revendication 13 pour effectuer tous les stades d'un procédé suivant l'une des revendications 1 à 11, lorsque les parties de programme sont réalisées par l'unité informatique de l'appareil médical suivant la revendication 13.
